# EUROPEAN PATENT APPLICATION

(11) **EP 3 342 690 A1**
(43) Date of publication of application: **04.07.2018**
(21) Application number: 17206568.2
(22) Date of filing: 12.12.2017
(51) Int. Cl.: B62J 1/26, B62J 1/00

(54) **BICYCLE SADDLE**

(30) Priority: 29.12.2016 TW 105143924
(71) Applicant: Velo Enterprise Co., Ltd., Dajia Township, Taichung County 437 (TW)
(72) Inventor: YU, Tsai-Yun, 437 TAICHUNG CITY (TW)
(74) Representative: Becker Kurig Straus

(57) **Abstract**

A bicycle saddle (10) comprises a load-bearing shell (20) having an upper surface (202) and an underside, a padding (30) disposed on the upper surface (202) of the shell (10), and a cover (40) covering over the padding (30) and thereby providing a seating surface for a bicycle rider to sit on. The padding (30) comprises a supporting layer (50) made of a first plastic foam material and disposed on the upper surface (202) of the shell (20), and a pressure-bearing layer (70) made of a second plastic foam material having a spring-back rate being lower than that of the first plastic foam material, which is arranged between the cover (40) and the supporting layer (50).

## Description

### BACKGROUND OF THE INVENTION

### 1. Field of the Invention

The present invention relates to a saddle for bicycles and the like.

### 2. Description of the Related Art

For improving the riding comfort, U.S. Patent No. 5,108,076 disclosed a bicycle saddle having not only an elastic layer made of plastic foam disposed between a shell and a covering but a gel layer arranged between the elastic layer and the covering. Such a design should have the expected riding comfort, however, there exists some drawbacks. For example, the gel layer must be arranged in a region corresponding to the rider's ischium and crotch and controlled at a specific thickness, otherwise, the saddle will be too soft, which will lead to excessive relative movement between the rider's buttocks, crotch and the saddle while riding, so that the purpose of improving comfort cannot be achieved properly.

### SUMMARY OF THE INVENTION

The present invention is proposed to solve the aforementioned drawbacks of prior art. Accordingly, a bicycle saddle, according to one aspect of the present invention, comprises a load-bearing shell having an upper surface and an underside, a padding disposed on the upper surface of the shell, and a cover covering over the padding and thereby providing a seating surface for a bicycle rider to sit on. The padding comprises a supporting layer made of a first plastic foam material and disposed on the upper surface of the shell, and a pressure-bearing layer made of a second plastic foam material having a spring-back rate being lower than that of the first plastic foam material, which is arranged between the cover and the supporting layer.

In more detail, the pressure-bearing layer of the padding may be made of a temper foam material sold on the market. The temper foam material is often referred to as "viscoelastic" polyurethane foam, or low-resilience polyurethane foam. Therefore, when the bicycle saddle of the present invention is pressurized by the rider's body during riding, the pressure-bearing layer thereof is molded to conform to the contour of the rider's body, thereby the pressure on the pressure-bearing layer can uniformly distribute to the entire contact surface thereof, helping relieve pressure points, preventing pressure sores, etc., and thus improving riding comfort of the rider.

### BRIEF DESCRIPTION OF THE DRAWINGS

A full and enabling disclosure of the present disclosure including the best mode thereof, enabling one of ordinary skill in the art to carry out the disclosure, is set forth in greater detail in the following description, including reference to the accompanying drawing in which:

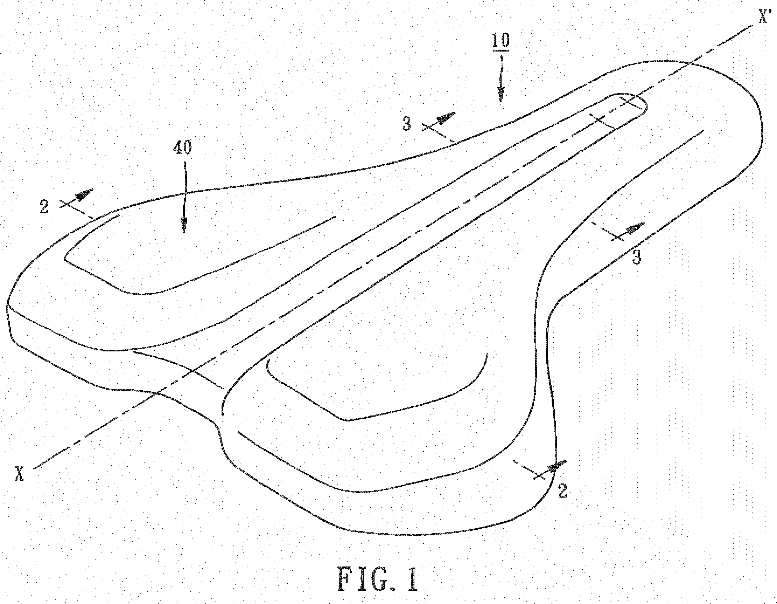

FIG. 1 is a perspective view of a first embodiment of the present invention;

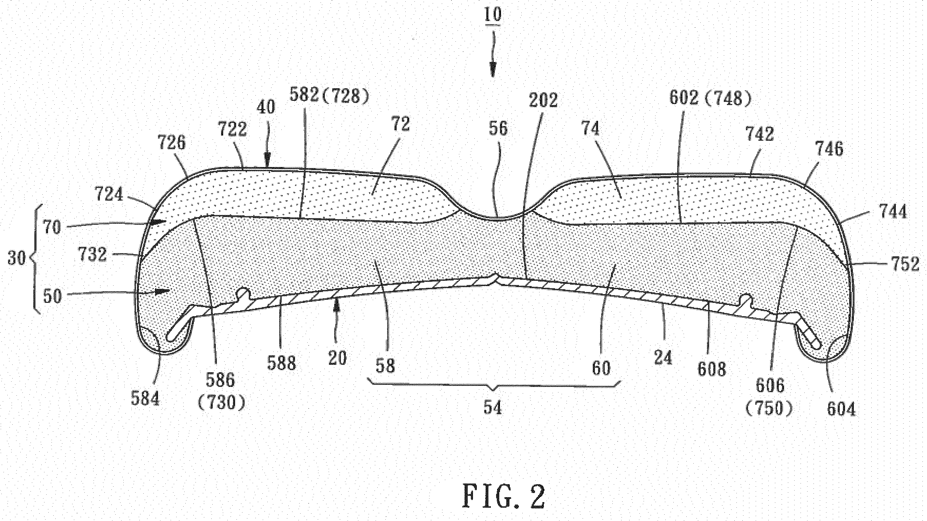

FIG. 2 is a sectional view of the first embodiment of the present invention along line 2--2 in FIG. 1;

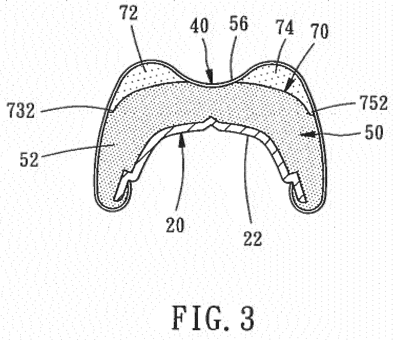

FIG. 3 is a sectional view of the first embodiment of the present invention along line 3--3 in FIG. 1; and
FIG. 4 is a perspective view of the padding of the first embodiment of the present invention.

### DETAILED DESCRIPTION OF PREFERRED EMBODIMENTS

With reference to the cited drawings, a bicycle saddle according to the present invention is generally designated by the reference numeral 10. The bicycle saddle 10 comprises a load-bearing shell 20, a padding 30 disposed on the shell 20, and a cover 40 covering tightly over the padding 30 and thereby providing a seating surface for a bicycle rider to sit on.

The shell 20 is molded by rigid or semi-rigid plastic materials in a monocoque construction. In this embodiment, the shell 20 has a relatively narrow anterior portion 22, which becomes progressively narrower towards its front end. The shell 20 further comprises a relatively wide posterior portion 24 to form the seat, and a longitudinal axis X-X' extending from the front end of the narrow anterior portion 22 to the rear end of the wide posterior portion 24.

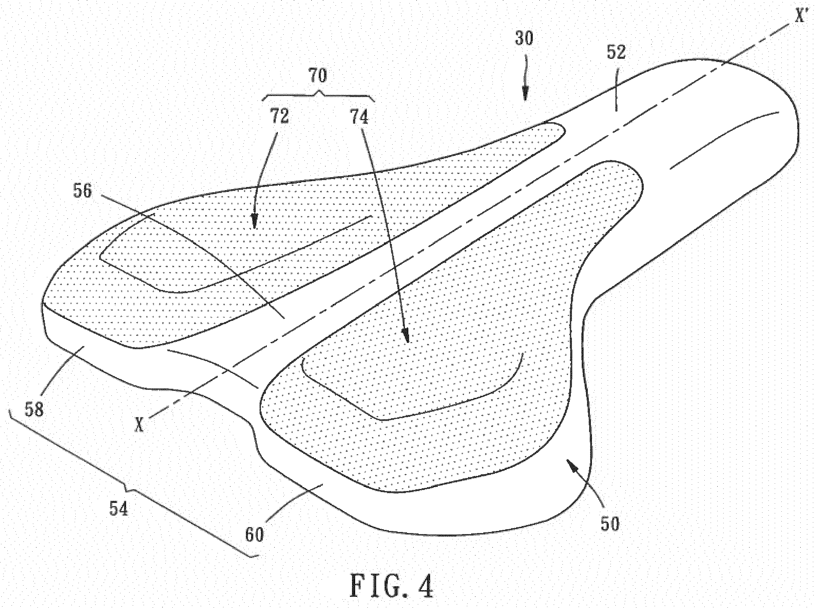

The padding 30, as shown in FIG. 4, comprises a supporting layer 50 and a pressure-bearing layer 70. The supporting layer 50 is made of a PU foam material with a high spring-back rate and disposed on an upper surface 202 of the shell 20. The pressure-bearing layer 70 is made of a temper foam material with a slow spring-back rate, which is arranged between the cover 40 and the supporting layer 50. In this embodiment, the supporting layer 50 has a first predetermined average thickness, and the pressure-bearing layer 70 has a second predetermined average thickness, which is less than the first predetermined average thickness.

In more detail, the supporting layer 50 includes a front supporting portion 52 bonded onto the relatively narrow anterior portion 22 of the shell 20 and a rear supporting portion 54 bonded onto the relatively wider posterior portion 24 of the shell 20. Preferably, the rear supporting portion 54 has a long depression 56 extending along the longitudinal axis X-X' of the shell 20 to a part of front supporting portion 52. The depression 56 divides the rear supporting portion 54 into a right rear supporting portion 58 and a left rear supporting portion 60. The right rear supporting portion 58 has a first right upper surface 582, a first right side surface 584, a first right convex arc surface 586 connecting the first right upper surface 582 and the first right side surface 584, and a first right under surface 588 being bonded onto to the upper surface 202 of the shell 20. The left rear supporting portion 60 has a first left upper surface 602, a first left side surface 604, a first left convex arc surface 606 connecting the first left upper surface 602 and the first left side surface 604, and a first left under surface 608 being bonded onto the upper surface 202 of the shell 20.

The pressure-bearing layer 70 includes a right pressure-bearing portion 72 and a left pressure-bearing portion 74 which are shaped symmetrically. The right pressure-bearing portion 72 has a second right upper surface 722, a second right side surface 724, a second right convex arc surface 726 connecting the second right upper surface 722 and the second right side surface724, and a second right under surface 728 bonded onto the first right upper surface 582 of the right rear supporting portion 58. The left pressure-bearing portion 74 has a second left upper surface 742, a second left side surface 744, a second left convex arc surface 746 connecting the second left upper surface 742 and the second left side surface744, and a second left under surface 748 bonded onto the first left upper surface 602 of the left rear supporting portion 60. Each of second right and left under surfaces 728, 748 has a concave arc portion 730,750 respectively bonded onto each of the first right and left convex arc surfaces 586, 606.

In addition, each of the second right and left side surfaces 724,744 has respectively a first lower end, each of second right and left convex arc surfaces 726, 746 has respectively a second lower end joining to the first lower end so as to define an outer edge 732,752. Each of outer edges 732,752 is respectively connected with the upper end of each of the first right and left side surfaces 584, 604 so that the saddle 10 will have a smooth outer side surface.

The cover 40 is generally made of cloth, leather, artificial leather or other synthetic materials and covers tightly over the whole upper surface of the pressure-bearing layer 70 of the padding 30.

It can be seen from the foregoing disclosures that the bicycle saddle 10 embodied according one aspect of the present invention includes the padding 30 combined by the supporting layer 50 and the pressure-bearing layer 70. The supporting layer 50 is made of a PU foam material with a high spring-back rate thus on the one hand providing a suitable elasticity of the saddle 10 and on the other hand also providing a suitable support of the saddle 10. And, it must be mentioned that the pressure-bearing layer 70 is made of a temper foam material such that when the bicycle saddle 10 is pressurized by the rider's body during riding, the pressure-bearing layer 70 is molded to conform to the contour of the rider's body, thereby the pressure on the pressure-bearing layer 70 can uniformly distribute to the entire contact surface thereof, helping relieve pressure points, preventing pressure sores, etc., and thus improving riding comfort of the rider.

## Claims

1. A bicycle saddle (10), comprising:
a load-bearing shell (20) having an upper surface (202) and an underside;
a padding (30) disposed on the upper surface (202) of the shell; and
a cover (40) covering over the padding (30) and thereby providing a seating surface for a bicycle rider to sit on;
wherein the saddle (10) is **characterized in that** the padding (30) comprises:
a supporting layer (50) made of a first plastic foam material and disposed on the upper surface (202) of the shell (20); and
a pressure-bearing layer (70) made of a second plastic foam material with a spring-back rate being lower than that of the first plastic foam material, the pressure-bearing layer (70) arranged between the cover (40) and the supporting layer (50).

2. The bicycle saddle (10) of claim 1, wherein the first plastic foam material is a PU foam material.

3. The bicycle saddle (10) of claim 2, wherein the second plastic foam is a temper foam material.

4. The bicycle saddle (10) of claim 1, wherein the supporting layer (50) has a first predetermined average thickness, and the pressure-bearing layer (70) has a second predetermined average thickness, which is less than the first predetermined average thickness.

5. The bicycle saddle (10) of claim 1, wherein the supporting layer (50) has a first upper surface (582) (602), a first side surface (584) (604), a first convex arc surface (586) (606) connecting the first upper surface (582) (602) and the first side surface (584) (604), and a first under surface (588) (608) attaching to the upper surface (202) of the shell (20).

6. The bicycle saddle of claim 5, wherein the pressure-bearing layer (70) has a second upper surface (722) (742), a second side surface (724) (744), a second convex arc surface (726) (746) connecting the second upper surface (722) (742) and the second side surface (724) (744), and a second under surface (728) (748) attaching to the first upper surface (582) (602) of the supporting layer (50), the second under surface (728) (748) having a concave arc portion (730) (750) for receiving the first convex arc surface (586) (606) of the supporting layer (50).

7. The bicycle saddle (10) of claim 6, wherein the second side surface (724) (744) of the pressure-bearing layer (70) has a first lower end, the second under surface (728) (748) of the pressure-bearing layer (70) has a second lower end joining to the first lower end of the second side surface (724) (744) so as to define an outer edge of the pressure-bearing layer (70), which is connected with an upper end of the first side surface (584) (604) of the supporting layer (50).

8. The bicycle saddle (10) of claim 1, wherein the shell (20) includes a longitudinal axis, a relatively narrow anterior portion (22) and a relatively wider posterior portion (24).

9. The bicycle saddle (10) of claim 8, wherein the supporting layer (50) includes a front supporting portion (52) arranged over the relatively narrow anterior portion (22) of the shell (20) and a rear supporting portion (54) arranged over the relatively wider posterior portion (24) of the shell (20).

10. The bicycle saddle (10) of claim 9, wherein the rear supporting portion (54) has a depression (56) extending along the longitudinal axis of the shell (10) to a part of the front supporting portion (52) and dividing the rear supporting portion (54) into a right rear supporting portion (58) and a left rear supporting portion (60).

11. The bicycle saddle (10) of claim 10, wherein the pressure-bearing layer (70) includes a right pressure-bearing portion (72) disposed over the right rear supporting portion (58), and a left pressure-bearing portion (74) disposed over the left rear supporting portion (60).

12. The bicycle saddle (10) of claim 11, wherein the right rear supporting portion (58) has a first upper surface (582), a first side surface (584), a first convex arc surface (586) connecting the first upper surface (582) and the first side surface (584), and a first under surface (588) attaching to the upper surface (202) of the shell (20), and the left rear supporting portion (60) has a first upper surface (602), a first side surface (604), a first convex arc surface (606) connecting the first upper surface (602) and the first side surface (604), and a first under surface (608) attaching to the upper surface (202) of the shell (20).

13. The bicycle saddle (10) of claim 12, wherein the right pressure-bearing portion (72) has a second upper surface (722), a second side surface (724), a second convex arc surface (726) connecting the second upper surface (722) and the second side surface (724), and a second under surface (728) attaching to the first upper surface (582) of the right rear supporting portion (58), the second under surface (728) having a concave arc portion (730) for receiving the first convex arc surface (586) of the right rear supporting portion (58), and the left pressure-bearing portion (74) has a second upper surface(742), a second side surface (744), a second convex arc surface (746) connecting the second upper surface (742) and the second side surface (744), and a second under surface (748) attaching to the first upper surface (602) of the left rear supporting portion (60), the second under surface (748) having a concave arc portion (750) for receiving the first convex arc surface (606) of the left rear supporting portion (60).

14. The bicycle saddle (10) of claim 13, wherein the second side surface (724) of the right pressure-bearing portion (72) has a first lower end, the second under surface (728) of the right pressure-bearing portion (72) has a second lower end joining to the first lower end of the second side surface (724) so as to define an outer edge of the right pressure-bearing portion (72), which is connected with an upper end of the first side surface (584) of the right rear supporting portion (58), and the second side surface (744) of the left pressure-bearing portion (74) has a first lower end, the second under surface (748) of the left pressure-bearing portion (74) has a second lower end joining to the first lower end of the second side surface (744) so as to define an outer edge of the left pressure-bearing portion (74), which is connected with an upper end of the first side surface (604) of the left rear supporting portion (60).
